# EUROPEAN PATENT APPLICATION

(11) **EP 4 527 848 A1**
(43) Date of publication of application: **26.03.2025**
(21) Application number: 23807876.0
(22) Date of filing: 16.05.2023
(51) Int. Cl.: C07K 14/00, A61K 47/42, G01N 33/68, C12N 15/70

(54) **POLYPEPTIDE THAT PASSES THROUGH BLOOD-BRAIN BARRIER, AND USE THEREOF**

(30) Priority: 16.05.2022 KR 20220059758
(71) Applicant: Korea University Research and Business Foundation, Seoul 02841 (KR); Medi&Gene Inc., Seoul 02841 (KR)
(72) Inventor: SHIN, Min-Jeong, Seoul 06285 (KR); CHUNG, In Hyeok, Goyang-si, Gyeonggi-do 10521 (KR); PARK, Chan, Uijeongbu-si, Gyeonggi-do 11627 (KR)
(74) Representative: Reddie & Grose LLP
(86) International application number: PCT/KR2023/006613
(87) International publication number: WO 2023/224357

(57) **Abstract**

The present invention relates to a polypeptide that passes through a blood-brain barrier or cerebrovascular barrier and a use of the polypeptide for material delivery, and the polypeptide can deliver a material that naturally cannot pass through the blood-brain barrier such as protein into brain tissue.

## Description

### TECHNICAL FIELD

The present invention relates to a polypeptide that passes through a blood-brain barrier or cerebrovascular barrier and a use of the polypeptide for material delivery.

### BACKGROUND ART

A Blood-Brain Barrier (BBB) or cerebrovascular barrier is a barrier that separates cerebrospinal fluid and blood. It has high selective permeability and plays a role of isolating major regulatory centers of the body from pathogens that can be transported from blood, such as bacterial and the like, and potential hazardous materials in blood.

The blood-brain barrier plays a role of passively allowing glucose, essential amino acids, electrolytes and the like to pass through it through endothelial cells, but preventing metabolites, toxins, and drugs in blood from entering brain cells. However, fat-soluble materials such as water, air and carbon dioxide can freely pass through the barrier, so alcohol, nicotine, anesthetics, and the like affect the brain.

Such selective permeability of the blood-brain barrier is a major obstacle to pharmacological treatment of brain and central nervous system (CNS) diseases such as Alzheimer' s disease, Parkinson' s disease, bacterial and viral infection and cancer. That is because many therapeutic agents for treating brain and central nervous system (CNS) diseases and disorders are hydrophilic and thus they cannot directly pass through the blood-brain barrier.

### DISCLOSURE

### TECHNICAL PROBLEM

Under the above circumstance, the present inventors have studied to discover a novel blood-brain barrier permeable peptide, and have confirmed that fusion proteins of 5 kinds of novel peptides and GST passed through the blood-brain barrier through in vivo experimental results.

Accordingly, an object of the present invention is to provide a novel blood-brain barrier permeable peptide and a use for brain delivery of a target material of the peptide.

### TECHNICAL SOLUTION

In order to achieve the above object, one aspect of the present invention provides a blood-brain barrier permeable peptide comprising an amino acid sequence of SEQ ID NO: 3 or SEQ ID NO: 5.

According to one specific embodiment of the present invention, the blood-brain barrier permeable peptide comprising the amino acid sequence of SEQ ID NO: 3 may be selected from the group consisting of SEQ ID NOs: 1 to 3, and the blood-brain barrier permeable peptide comprising the amino acid sequence of SEQ ID NO: 5 may consist of an amino acid sequence of SEQ ID NO: 4 or SEQ ID NO: 5.

In the present invention, the blood-brain barrier permeable peptide comprising the amino acid sequence of SEQ ID NO: 3 may comprise an amino acid sequence having sequence homology of 70% or more, preferably, 80% or more, more preferably, 90% or more, further more preferably, 95% or more, most preferably, 98% or more to the amino acid sequence selected from the group consisting of SEQ ID NOs: 1 to 3.

In the present invention, the blood-brain barrier permeable peptide comprising the amino acid sequence of SEQ ID NO: 5 may comprise an amino acid sequence having sequence homology of 70% or more, preferably, 80% or more, more preferably, 90% or more, further more preferably, 95% or more, most preferably, 98% or more to the amino acid sequence of SEQ ID NO: 5.

On the other hand, the blood-brain barrier permeable peptide may be used by being fused with another cell membrane permeable peptide, and the examples of the cell membrane permeable peptide include Penetratin, TAT peptide, Pep-1 peptide, S413-PV, Magainin 2 or Buforin 2.

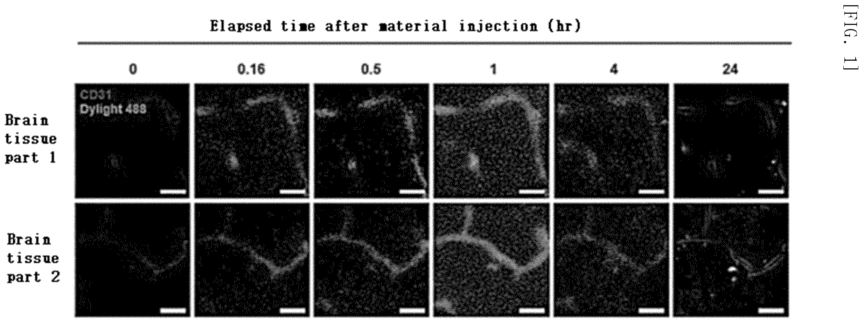

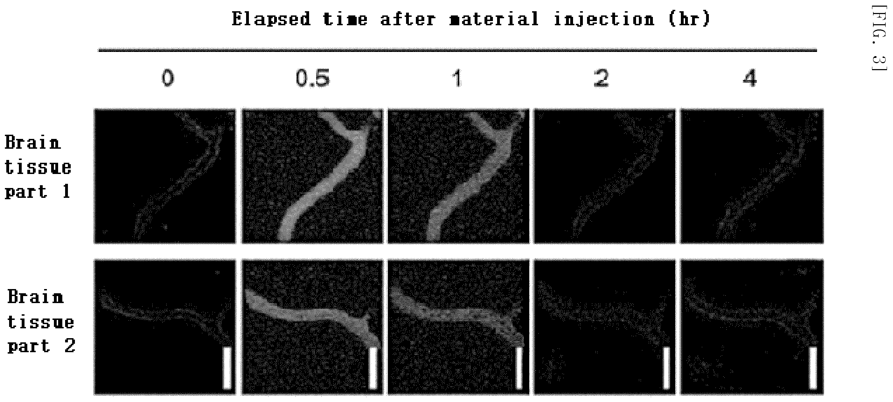

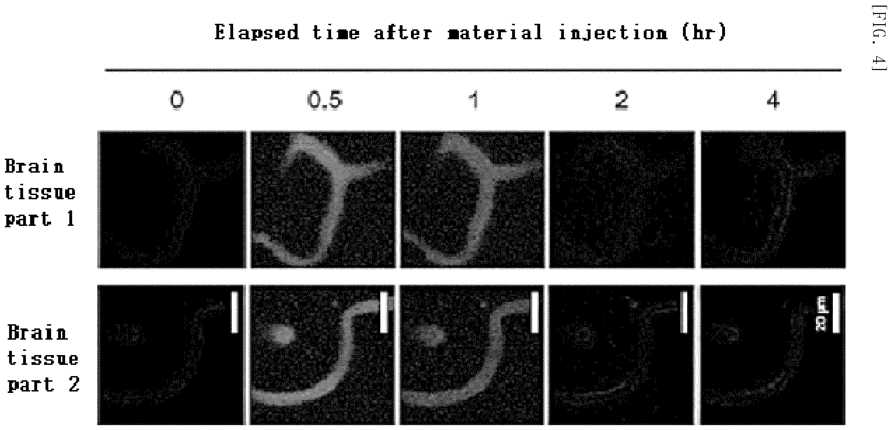

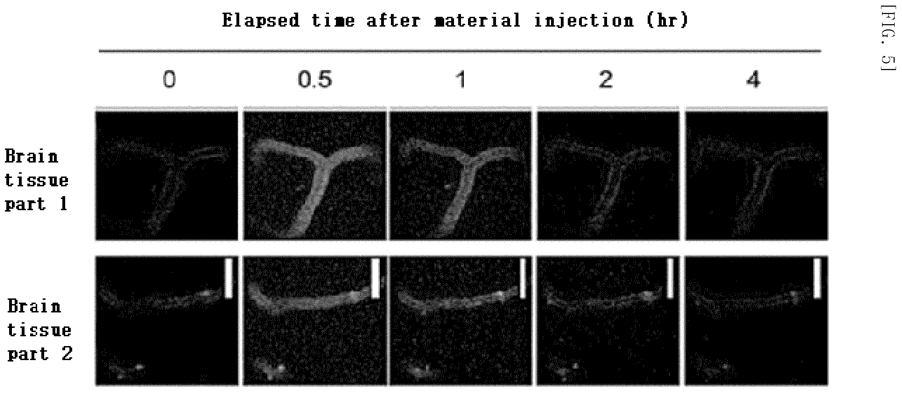

The present inventors have confirmed that the fusion proteins of the discovered blood-brain barrier permeable peptides and GST permeated the blood-brain barrier of mice (FIGs. 1 to 5).

Accordingly, another aspect of the present invention provides a system for brain delivery of a target material comprising a blood-brain barrier permeable peptide; and a target material bound to one terminal of the blood-brain barrier permeable peptide.

The target material may be selected from the group consisting of compounds, proteins, glycoproteins, peptides, nucleotides, nucleic acids, carbohydrates, lipids, glycolipids, nanoparticles, liposomes, drugs, lipid-based formulations, viruses, quantum dots and fluorochromes.

In the present invention, the blood-brain barrier permeable protein and target material may be linked by a method for binding a protein and a target material known in the art, and for example, it may be linked, for example, by a covalent bond, a chemical bond, a peptide bond, and the like.

As described above, the present inventors have confirmed that the fusion proteins of the blood-brain barrier permeable peptides and GST permeated the blood-brain barrier, and thus, when a protein (target protein) is used as a target material, it may be introduced in the form of a fusion protein of a blood-brain barrier permeable protein and a target protein into brain tissue.

On the other hand, when a therapeutic agent or a diagnostic agent of brain or central nervous system diseases is used, the system for brain delivery of a target material can be used as a pharmaceutical composition for treating or preventing brain or central nervous system diseases, or a composition for diagnosing brain or central nervous system diseases.

For example, the present invention can contrast the distribution of the target material in cerebral parenchyma through PET or inhibit aggregation, by linking a substance inhibiting aggregation by selectively combining to various target materials of which aggregation increases in degenerative brain diseases, such as alpha-synuclein, amyloid beta, tau, S0D1 (Superoxide dismutase1), TDP-43, and the like, with the blood-brain barrier permeable peptide, and then injecting that into the body.

The brain or central nervous system diseases include generally called degenerative neurological diseases, and include Alzheimer' s dementia, Parkinson' s disease, frontotemporal dementia, Lewy dementia, and Huntington' s disease, and the like.

The composition for diagnosing brain or central nervous system diseases may be provided as implemented in the form of a kit for predicting or diagnosing the degree of risk of brain or central nervous system diseases. This kit may not only comprise an agent capable of detecting a blood-brain barrier permeable peptide, but also comprise one or more kinds of other component compositions, solutions, or devices suitable for the analysis method.

The pharmaceutical composition for treating or preventing brain or central nervous system diseases may be orally administered or parenterally administered (for example, intravenous, subcutaneous, intraperitoneally or locally applied) according to a targeted method, and when formulated, it is prepared using a diluent or excipient such as a filler, extender, binder, wetting agent, disintegrating agent, surfactant, or the like.

Solid preparations for oral administration include tablets, pills, powders, granules, capsules, troches, and the like, and these solid preparations are prepared by mixing at least one or more excipients, for example, starch, calcium carbonate, sucrose or lactose or gelatin or the like to the active ingredient. In addition, in addition to the simple excipient, lubricants such as magnesium stearate talc are used. Suspensions, oral liquids, emulsions or syrups or the like correspond to liquid preparations for oral administration, and in addition to water and liquid paraffin, which are commonly used simple diluents, various excipients, for example, wetting agents, sweeteners, flavoring agents, preservatives and the like may be comprised.

Sterilized aqueous solutions, non-aqueous solvents, suspensions, emulsions, lyophilized preparations, suppositories and the like are included in preparations for parenteral administration. As the non-aqueous solvents and suspensions, propylene glycol, polyethylene glycol, vegetable oils such as olive oil, injectable esters such as ethyl oleate and the like may be used. As a base compound of the suppositories, witepsol, macrogol, tween 61, cacao butter, laurin butter, glycerol, gelatin, and the like may be used.

The pharmaceutical composition of the present invention is administered in a pharmaceutically effective amount. In the present invention, the 'pharmaceutically effective amount' means an amount sufficient to treat diseases with a reasonable benefit/risk ratio applicable to medical treatment, and the effective dose level may be determined depending on elements including the type and severity of the patient' s disease, the activity of the drug, the sensitivity to the drug, the administration time, administration route and excretion rate, the treatment period, concurrently used drugs, and other elements well known in the medical field. For example, the pharmaceutical composition of the present invention may be administered by being divided once a day or three times a day at a dose of 0.1 mg/kg to 200 mg/kg, preferably, 1 mg/kg to 50 mg/kg. The dose does not limit the scope of the present invention in any way. However, it may be increased or decreased depending on the administration route, the severity of obesity, gender, body weight, age, and the like, so it does not limit the scope of the present invention in any way.

Other aspect of the present invention provides a polynucleotide encoding the blood-brain barrier permeable peptide.

The term used in the present description, 'polynucleotide' is a polymer of deoxyribonucleotide or ribonucleotide present in a single-stranded or double-stranded form. It encompasses RNA genomic sequences, DNA (gDNA and cDNA) and RNA sequence transcribed therefrom, and unless otherwise specifically mentioned, it includes analogues of natural polynucleotides.

In the present invention, the polynucleotide includes not only nucleotide sequences encoding cell membrane permeable peptides, but also sequences complementary to the sequences. The complementary sequences include not only completely complementary sequences, but also substantially complementary sequences. In addition, the polynucleotide sequences may be modified, and modification includes addition, deletion, or non-conservative substitution or conservative substitution of nucleotides.

Other aspect of the present invention provides a recombinant vector comprising a polynucleotide sequence encoding the blood-brain barrier permeable protein.

The term used in the present description, 'vector' refers to a means to express a target gene in a host cell. For example, viral vectors such as plasmid vectors, cosmid vectors, and bacteriophage vectors, adenovirus vectors, retrovirus vectors, and adeno-associated virus vectors, may be included, but not limited thereto. Vectors which can be used as the recombinant vector may be produced by engineering plasmids (for example, pSC101, pGV1106, pACYC177, ColE1, pKT230, pME290, pBR322, pUC8/9, pUC6, pBD9, pHC79, pIJ61, pLAFR1, pHV14, pGEX series, pET series and pUC19, etc.), phages (for example, λgt4λB, λ-Charon, λ Δ z1 and M13, etc.), or viruses (for example, CMV, SV40, etc.), often used in the art.

The recombinant vector may comprise the polynucleotide sequence and a promoter operatively linked to the polynucleotide sequence.

The term used in the present description, 'operatively linked' means functional binding between a regulatory sequence of nucleotide expression (for example, promoter sequence) and another nucleotide sequence, and thereby, the regulatory sequence regulates transcription and/or translation of another nucleotide sequence.

According to one specific embodiment of the present invention, when a protein is used as a target material, by introducing a blood-brain barrier permeable protein and a polynucleotide sequence encoding a target protein into a vector together, it may be expressed in the form of a fusion protein.

Therefore, the recombinant vector may comprise a tag sequence that facilitates purification of a fusion protein, for example, a consecutive histidine codon, a maltose binding protein codon, and a Myc codon, and the like. In addition, the recombinant vector may further comprise a fusion partner to increase solubility of the fusion protein. Furthermore, it may comprise a sequence specifically cleaved by an enzyme to remove unnecessary portions, an expression regulatory sequence, and a marker or reporter gene to confirm intracellular delivery.

### ADVANTAGEOUS EFFECTS

Materials that cannot naturally pass though the blood-brain barrier can be effectively delivered into brain tissue, when the blood-brain barrier permeable polypeptide of the present invention is used.

### BRIEF DESCRIPTION OF THE DRAWINGS

FIG. 1 is the result of confirming the blood-brain barrier permeability of the polypeptide in brain tissue over time, after labeling a fluorescent material to the protein in which GST and the polypeptide of SEQ ID NO: 1 are fused and administering it to mice (red: blood vessels, green: polypeptide).
FIG. 2 is the result of confirming the blood-brain barrier permeability of the polypeptide in brain tissue over time, after labeling a fluorescent material to the protein in which GST and the polypeptide of SEQ ID NO: 2 are fused and administering it to mice (red: blood vessels, green: polypeptide).
FIG. 3 is the result of confirming the blood-brain barrier permeability of the polypeptide in brain tissue over time, after labeling a fluorescent material to the polypeptide of SEQ ID NO: 3 and administering it to mice (red: blood vessels, green: polypeptide).
FIG. 4 is the result of confirming the blood-brain barrier permeability of the polypeptide in brain tissue over time, after labeling a fluorescent material to the polypeptide of SEQ ID NO: 4 and administering it to mice (red: blood vessels, green: polypeptide).
FIG. 5 is the result of confirming the blood-brain barrier permeability of the polypeptide in brain tissue over time, after labeling a fluorescent material to the polypeptide of SEQ ID NO: 5 and administering it to mice (red: blood vessels, green: polypeptide).

### MODE FOR INVENTION

Hereinafter, one or more embodiments will be described in more detail through examples. However, these examples are intended to illustratively describe one or more embodiments, but the scope of the present invention is not limited by these examples.

### Example 1: Preparation of peptides

Peptides were prepared as follows, and the peptide sequences were written in Table 1.

**[Table1]**

| SEQ ID NO: | Peptide sequences |
|---|---|
| 1 | GSDQSENVDRGAGSIREAGGAFGKREQAEEERYFRAQSREQLAALKKHHEEEIVHHKKEIERLQKE IER HKQKIKMLKHDD |
| 2 | GSDQSENVDRGAGSIREAGGAFGKREQAEEERYFRAQSREQLAALKK |
| 3 | VDRGAGSIREAGGAFGKREQAEEERYFR |
| 4 | GSDQSENVDRG |
| 5 | GSDQSEN |

In order to evaluate whether permeable peptides could be used as a drug delivery system, GST which could not pass through a blood-brain barrier naturally was conjugated to the N-terminal of a peptide corresponding to SEQ ID NO: 1 or 2.

Specifically, a recombinant vector was created by inserting the peptide sequence of SEQ ID NO: 1 or 2 and a nucleotide sequence encoding GST into pGEX vector system, and this recombinant vector was introduced to E. coli strain BL21(DE3) by transformation. The strain was cultured in an LB medium at 26°C for 3 hours, and then IPTG (isopropyl-β-D-thiogalactopyranoside) of 1 mM was treated, and it was cultured at 18°C for 12 hours.

After that, the cultured solution was recovered to recover cells by centrifugation, and the cells were sonicated. Fusion proteins of GST+peptides were obtained by affinity chromatography. For the obtained fusion proteins, the salt concentration was adjusted for 2~3 days using PBS (pH 7.4) buffer solution and the purity was increased.

The peptides of SEQ ID NOs: 3, 4 and 5, except for SEQ ID NOs: 1, 2, were synthesized, and after confirming that the purity was 95% or higher, they were used for subsequent experiments.

### Example 2: Evaluation of BBB permeability of peptides

Whether the peptides of the present invention could permeate the blood-brain barrier in a biological system was confirmed as follows.

In order to trace peptides in vivo, Dylight488 exhibiting fluorescent signals was combined to the fusion protein of GST+peptide or the peptide of SEQ ID NO: 3 to 5 produced in Example 1. Each fusion protein or peptide sample in which a fluorescent material was combined was injected into mice (systematic name: C57BL/6N, 8 weeks old) known to show metabolic functions similar to the human body, and the presence of the sample in brain tissue and blood vessel areas was observed.

The movement of the mice was limited, and to prevent inappropriate result interpretation according to that, anesthesia (isoflurane) was performed. In order to distinguish brain tissue and blood vessels, blood vessels were stained with anti-CD31 and FSD fluor 647 compounds, and for tissue imaging, skin tissue on the skull was incised, and a cranial window was attached to the incision site to obtain fixed images. After that, tissue images immediately before sample injection (0 hr) were photographed with a fluorescence microscope (IVM-CM Confocal & Two-photon convertible microscopy), and the sample (10 mg/kg) was intravenously injected to observe the fluorescent signal pattern over time.

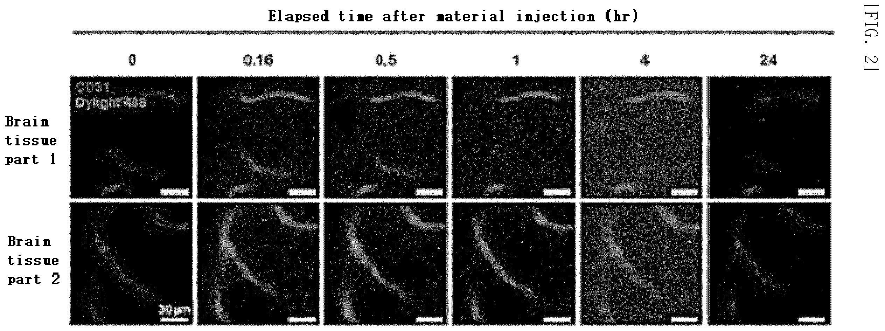

As a result, for Peptides 1 and 2, the fluorescent signals could be observed in blood vessels in brain tissue from the time of 0.16 hours (10 minutes) after injection, and it could be found that the fluorescent signals in the brain tissue was the highest at the time of 1 hour after injection for Peptide 1, and 4 hours after injection for Peptide 2 (FIG. 1 and FIG. 2).

For Peptides 3 to 5 all, the fluorescent signals could be observed in blood vessels in brain tissue from the time of 0.5 hours (30 minutes) after injection, and the fluorescent signals were the highest at the time of 1 hour after injection (FIGs. 1 to 5).

Through the results of FIGs. 1 to 5, it was confirmed that the peptides of the present invention could permeate the blood-brain barrier, and effectively deliver large materials such as GST into brain tissue.

## Claims

1. A blood-brain barrier permeable peptide comprising an amino acid sequence of SEQ ID NO: 3 or SEQ ID NO: 5.

2. The blood-brain barrier permeable peptide according to claim 1, wherein the blood-brain barrier permeable peptide comprising an amino acid sequence of SEQ ID NO: 3 is selected from the group consisting of SEQ ID NOs: 1 to 3.

3. The blood-brain barrier permeable peptide according to claim 1, wherein the blood-brain barrier permeable peptide comprising an amino acid sequence of SEQ ID NO: 5 consists of an amino acid sequence of SEQ ID NO: 4 or SEQ ID NO: 5.

4. A system for brain delivery of a target material comprising; the blood-brain barrier permeable peptide according to any one claim of claim 1 to claim 3; and a target material bound to one terminal of the blood-brain barrier permeable peptide.

5. The system for brain delivery of a target material according to claim 4, wherein the target material is selected from the group consisting of compounds, proteins, glycoproteins, peptides, nucleotides, nucleic acids, carbohydrates, lipids, glycolipids, nanoparticles, liposomes, drugs, lipid-based formulations, viruses, quantum dots and fluorochromes.

6. The system for brain delivery of a target material according to claim 4, wherein the blood-brain barrier permeable peptide and the target material are linked by a covalent bond.

7. A polynucleotide encoding the blood-brain barrier permeable peptide according to any one claim of claim 1 to claim 3.

8. A recombinant vector comprising the polynucleotide of claim 7.

9. A pharmaceutical composition for treating or preventing brain or central nervous system diseases, comprising the system for brain delivery of a target material according to any one claim of claim 4 to claim 6 as an active ingredient.

10. A pharmaceutical composition for diagnosing brain or central nervous system diseases, comprising the system for brain delivery of a target material according to any one claim of claim 4 to claim 6 as an active ingredient.
